Europäisches Patentamt

European Patent Office

Office européen des brevets

(11) Publication number: **0 278 941**
**A1**

## (12) EUROPEAN PATENT APPLICATION

(21) Application number: 88870009.3

(22) Date of filing: 25.01.88

(51) Int. Cl.4: **C 12 N 15/00**
C 12 N 1/18, C 12 P 21/02,
C 07 K 13/00, A 61 K 39/015

(30) Priority: 30.01.87 US 8791

(43) Date of publication of application:
17.08.88 Bulletin 88/33

(84) Designated Contracting States:
AT BE CH DE ES FR GB GR IT LI LU NL SE

(71) Applicant: **Smith Kline - RIT Société anonyme dite:**
**rue du Tilleul, 13**
**B-1320 Genval (Rixensart) (BE)**

(72) Inventor: **De Wilde, Michel**
**Avenue Gevaert, 136**
**B-1320 Genval (BE)**

**Gathoye, Anne-Marie**
**Rue Claire Fontaine, 5**
**B-6200 Gosselies (BE)**

(74) Representative: **Tasset, Gérard et al**
**SMITHKLINE - RIT rue de l'Institut, 89**
**B-1330 Rixensart (BE)**

Claims for the following Contracting States: ES + GR.
The microorganism(s) has (have) been deposited with The American Type Culture Collection under number(s) ATCC 20819, 20630.

(54) **Expression of the P. falciparum circumsporozoite protein by yeast.**

(57) This invention relates to the expression of the Plasmodium falciparum circumsporozoite protein by yeast and to a vaccine comprising an immunoprotective amount of such protein.

TZ.TE.NS,5|

Figure 4

EP 0 278 941 A1

Bundesdruckerei Berlin

## Description

EXPRESSION OF THE P. FALCIPARUM CIRCUMSPOROZOITE PROTEIN BY YEAST

## BACKGROUND OF THE INVENTION

This invention related to the expression of the P. falciparum circumsporozoite protein by yeast, and to a vaccine comprising an immunoprotective amount of such protein.

Numerous recent studies suggest that protective immunity in a sensitive host to infection by the sporozoite stage of a particular species of Plasmodium can be mediated via antibodies produced by such host to the circumsporozoite protein (CS protein) of the particular sporozoite. This invention enables the production of the CS protein of the human malaria parasite P. falciparum or an immunogenic derivative thereof by yeast by recombinant DNA techniques.

It is desirable to use yeast for the production of such P> falciparum CS protein for a number of reasons including the well known and reliable, fermentation characteristics of endotoxin when such CS protein is prepared in yeast rather than gram-negative bacterial cells such as E. coli.

Nussenzweig et al., U.S. Patent 4,466,917, propose a sporozoite polypeptide from Plasmodium falciparum, identified as the Pf-44 protein, and its cloning and expression in E. coli.

Sharma et al., Science, 228, 279-282 (1985), disclose the expression of a portion of the circumsporozoite protein (CS) of Plasmodium knowlesi by yeast employing an expression vector containing the 5' regulatory region of the yeast alcohol dehydrogenase I gene in place of the 5' upstream region of the P. knowlesi CS gene sequence.

New York University, PCT Application Publication Number WO 84-02922-A, published August 2, 1984, disclose cloning of a portion of the coding region for the P. knowlesi CS protein repeat unit and expression of beta-lactamase and beta-galactosidase fusions thereof in E. coli.

Kemp et al., WO84-02917-A, disclose cloning and expression in E. coli of a coding sequence for the CS protein, derived from the blood stage of P. falciparum.

Dame et al., Science, 225, 593 (1984), report cloning and expression of the CS protein of P. falciparum in E. coli. The protein is described as comprising about 412 amino acids with an approximate molecular weight of 44,000. It comprises 41 tandem repeats of a tetrapeptide. Synethetic 7-, 11- and 15- residue peptides derived from the repeat region bound to monoclonal antibodies raised against the CS protein.

Ellis et al., Nature, 302, 536 (1983), report expression of a beta-lactamase - P. knowlesi CS protein fusion in E. coli.

Weber et al., Molecular and Bacterial Parasitology, 15, 305-316 (1985), disclose use of a CS protein gene of a Brazilian strain of P. falciparum, cloned in E. coli, as a probe to analyze the structure of 17 other P. falciparum strains by nucleic acid hybridization, and conclude that the CS protein gene is highly conserved in P. falciparum and that malaria vaccine development with the CS protein is unlikely to be complicated by strain variation.

Arnot et al., Science, 230, 815-818 (1985), disclose the cloning of the CS protein of P. vivax using a probe derived from P. cynomolgi CS gene to isolate the homologous gene from a bacteriophage library of cloned P. vivax genomic DNA and determination of its entire coding sequence and conclude that the coding sequence of the CS protein of P. vivax is homologous to that of the CS gene of P. knowlesi but not P. falciparum.

Young et al., Science, 228, 958-962 (1985), disclose the expression of P. falciparum CS proteins in E. coli for potential use in a human malaria vaccine.

The Walter and Eliza Hall Institute of Medical Research, PCT Patent Application No. WO84/02917, published August 2, 1984, disclose artificially constructed polynucleotide sequences substantially corresponding to all or a portion of P. falciparum mRNA or genomic DNA; the peptide corresponding to such sequence and a method for production thereof; and a composition for stimulating immune responses against P. falciparum antigens in a mammal comprising such peptide.

Mazier et al., Science, 231, 156-159 (1986), disclose that antibodies raised in mice immunized with several recombinant and synthetic peptides of the circumsporozoite protein of P. falciparum were evaluated for protective activity in a human hepatocyte culture system.

Barr et al., "Modern Approaches to New Vaccines and Aids", page 22, Cold Spring Harbor, New York, September 3-14, 1986, disclose the expression by S. cerevisiae of a portion of the P. vivax CS protein coding sequence fused with a yeast alcohol dehydrogenase-2/glyceraldehyde-3-phosphate dehydrogenase hybrid promoter. Barr et al., disclose the use of a regulated hybrid promoter for expression of the P. vivax CS protein. The exact structure of this promoter is not disclosed. The CS gene sequence employed for fusion to the promoter lacks the C-terminal region which is an "anchor" region for insertion in the cell membrane. The exact boundary is not specified. Whether or not the gene sequence used in the construction also contains the signal sequence is also not disclosed.

## SUMMARY OF THE INVENTION

This invention relates to a recombinant DNA vector comprising a DNA sequence operatively linked to an expression control sequence, and, optionally, additionally comprising a replicon which is functional in yeast, wherein the DNA sequence contains the coding sequence of the circumsporozoite protein of Plasmodium falciparum.

This invention also relates to a yeast host cell transformed with a recombinant DNA vector, wherein said vector comprises a DNA sequence operatively linked to an expression control sequence, and, optionally, additionally comprises a replicon which is functional in said host cell, wherein the DNA sequence contains the coding sequence of the circumsporozoite protein of Plasmodium falciparum. This invention also relates to a method of preparing such a transformed host cell which comprises transforming a yeast host cell with such recombinant vector.

This invention also relates to a method for preparing circumsporozoite protein of Plasmodium falciparum, which comprises culturing a yeast host cell transformed with a recombinant DNA vector in appropriate culture media and isolating such protein from a culture lysate of such host, wherein said vector comprises a DNA sequence operatively linked to an expression control sequence, and, optionally, additionally comprises a replicon which is functional in said host cell, wherein the DNA sequence contains the coding sequence of the circumsporozoite protein of Plasmodium falciparum. This invention also relates to the protein so prepared, and to a vaccine comprising an immunoprotective quantity of such protein.

BRIEF DESCRIPTION OF THE DRAWING

Figure 1 is a flow chart illustrating preparation of pRIT10162.
Figure 2 is a restriction endonuclease map of plasmids pRIT10172, pRIT12570 and PRIT12569.
Figure 3 is a restriction endonuclease map of pRIT12290.
Figure 4 is a flow chart illustrating preparation of pRIT12570.
Figure 5 is a restriction endonuclease map of pMC200.

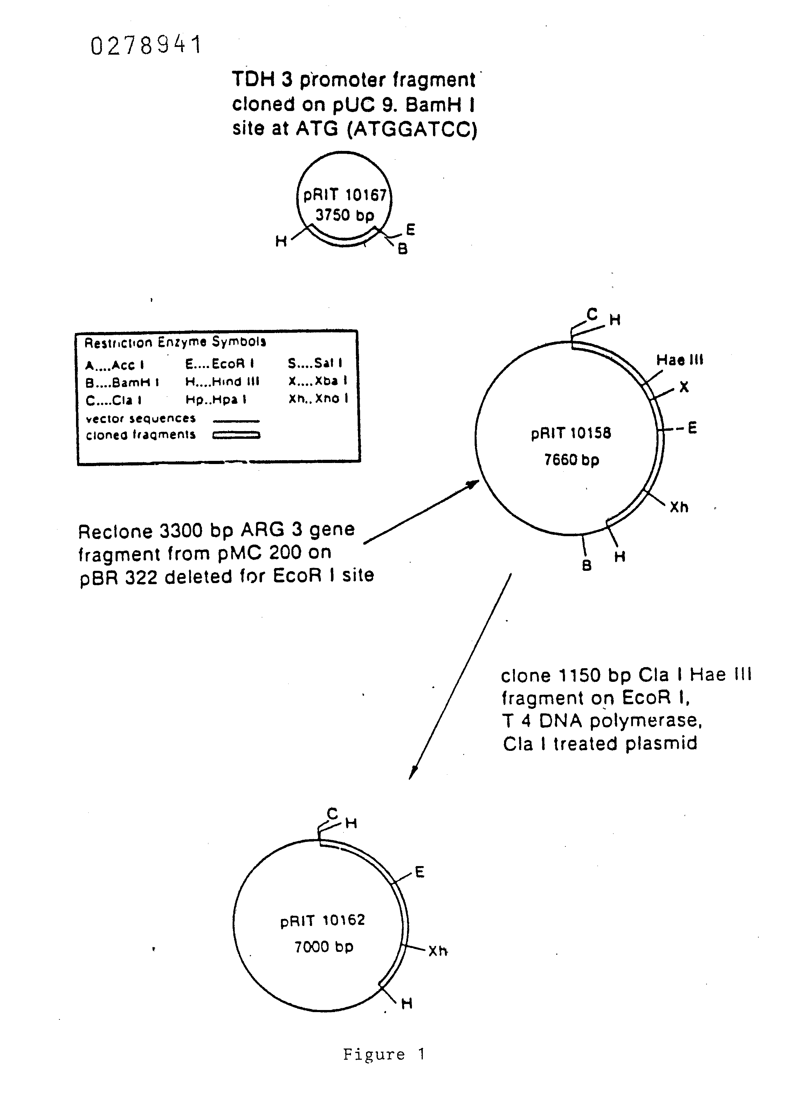

Figure 1A is a schematic restriction map illustrating the sequencing strategy of clone λmPfl.

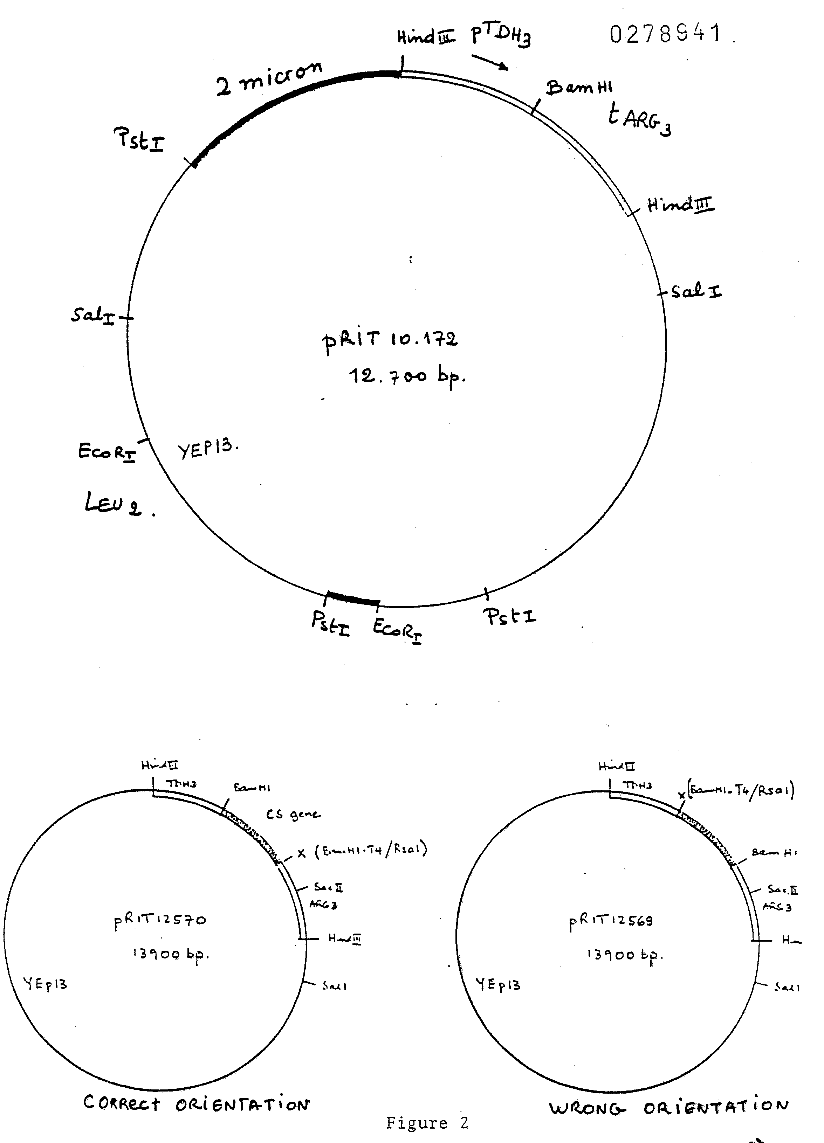

Figure 2A illustrates the nucleotide sequence of the CS protein gene from P. falciparum.

DETAILED DESCRIPTION OF THE INVENTION

By the term "the circumsporozoite protein of Plasmodium falciparum" is meant the immunodominant surface antigen on the sporozoite of Plasmodium falciparum ("CS protein").

The CS protein coding sequence of Plasmodium falciparum is disclosed by Dame et al., Science, 225, 593-599 (1984).

As used herein, the terms "circumsporozoite protein", "CS" or "CS" protein" will include both the full length circumsporozoite protein of Plasmodium falciparum as well as any immunogenic derivative thereof. By the term "immunogenic derivative" of the circumsporozoite protein of Plasmodium falciparum is meant (a) any derivative of the circumsporozoite protein of Plasmodium falciparum, such as derivative encoded by a subfragment of the full length CS protein coding sequence or a series of tandem subfragments, which retains protective immunogenic activity or (b) any protein derived from a modified coding sequence of the circumsporozoite protein of Plasmodium falciparum which retains protective immunogenic activity. Such immunogenic derivatives can be prepared by conventional techniques such as by the method of Botstein et al., Science, 229, 1193 (1985). Some immunogenic synthetic derivatives of the CS protein coding sequence of P. falciparum are disclosed in Ballou et al., Science, 228, 996-999 (1985).

The recombinant vector of this invention comprises a DNA molecule operatively linked to an expression control sequence, and optionally, additionally comprises a replicon which is functional in yeast, wherein the DNA molecule contains the coding sequence of the circumsporozoite protein of Plasmodium falciparum. By the term "replicon" is meant that minimum region of DNA which functions to maintain such recombinant vector in a host yeast microorganism transformed therewith. Such replicons are well known in the art. By the term "expression control sequence" is meant any region which regulates transcription of a structural gene's coding sequence. Such expression control sequences are well known in the art. Preferred expression control sequences include the yeast glyceraldehyde-3P-dehydrogenase gene (TDH3) promoter and the yeast ornithine carbamoyl transferase gene (ARG3) transcription termination region. Examples of other useful expression control sequences regions include those derived from yeast genes of the glycolytic pathway such as those coding for enolase, aldolase, and phosphoglycerate kinase; the yeast alcohol dehydrogenase gene; and, in general, genes involved in catabolism such as the arginase gene. Such vector can be prepared by conventional techniques such as by inserting the CS coding sequence of Plasmodium falciparum in phase into a vector already containing a replicon and expression control sequence in such a way that the DNA molecule is operatively linked to such expression control sequence. Examples of useful vectors into which to insert the CS coding sequence include the plasmid YEp13, which is capable of replication and maintenance in both E. coli and S. cerevisiae and is, therefore, known as a shuttle vector. Several other yeast vectors are known and available. Transformation with plasmid vectors containing autonomous replication sequences (replicons) function in yeast will normally result in extra-chromosomal maintenance of the DNA molecule of the invention as a plasmid. Such replicons are well known in the art. Transformation with plasmid vectors not containing replicons functional in yeast can result in incorporation of the DNA molecule into chromosomal DNA. Preferably the DNA molecule is ligated to a vector capable of expression in the yeast genus Saccharomyces, particularly S. cerevisiae.

Restriction of DNA to prepare DNA fragments used in the invention, ligation of such fragments to prepare recombinant DNA molecules used in the invention and insertion into microorganisms are carried out by known techniques such as techniques disclosed in the previously and subsequently cited references. Conditions are selected to avoid denaturation of the DNA and enzymes. For example, the pH is buffered to remain at about 7.0 to 11.0 and the temperature is kept below about 60°C. Preferably restriction is carried out at a temperature of about 30 to 40°C and ligation is carried out at about 0 to 10°C. Restriction enzymes and ligases used in carrying out this invention are commercially available and should be used in accordance with instructions included therewith. T4 DNA ligase is the preferred ligase.

The various fragments and final constructions may be joined together in accordance with conventional ways. In many cases, genes have been isolated and restriction mapped, as well as sequenced. To that extent, one can select the sequence of interest, such as the CS protein coding sequence, by restriction of the gene, employing further manipulation as necessary such as resection with Bal31, in vitro mutagenesis, primer repair, or the like, to provide a fragment of a desired size, including the desired sequence, and having the appropriate termini. Linkers and adapters can be used for joining sequences, as well as replacing lost sequences, where the restriction site is internal to the region of interest. The various fragments which are isolated, may be purified by electro-phoresis, electroeluted, ligated to other sequences, cloned, reisolated and further manipulated.

The use of expression control sequences for controlling transcription of the CS protein coding sequence, allows for growing the host cells to high density with no or low levels of expression of the CS protein coding sequence, then inducing expression by changing the environmental conditions, e.g., nutrient, temperature, etc.

For example, with the GAL4 regulatory region, the yeast cells could be grown in rich media with a glycerol-lactic acid combination to high density, e.g., mid or late log phase, followed by switching the carbon source to galactose. For PHO5 regulation one could grow the cells at high phosphate concentration to about 0.1 to 0.5 mM. For temperature sensitivity, one could grow the cells at 25° to 37°C and then change the temperature as appropriate by about 5° to 20°C. The host cells would have the regulatory system associated with the regulatory region employed.

Fusing of the CS protein coding sequence to the expression control can be accomplished by use of intermediate vectors, or, alternatively, the coding sequence can be inserted directly into a vector which contains the expression control sequence. Preferably, the CS protein coding sequence is positioned relative to the expression control sequence such that the protein synthesized by expression of the CS protein coding sequence is devoid of extraneous amino acid residues.

This invention also relates to a yeast host cell transformed with such recombinant vector and to a method of preparing such host. Such transformation is carried out by conventional techniques such as the method of Ito et al., J. Bacter; 153, 163-168 (1983). Preferred yeast host cells include those belonging to the genus Saccharomyces, particularly those belonging to the species Saccharomyces cerevisiae.

This invention also relates to a method for preparing Plasmodium falciparum circumsporozoite protein which comprises culturing the transformed yeast cells of this invention in appropriate culture media and isolating such protein from a culture lysate of such host cells. By "appropriate culture media" is meant that media which will enable the transformed host to survive and which will also enable such host to express the CS protein in recoverable quantity. It will be appreciated by one of skill in the art that the appropriate culture media to use will depend upon the host cell employed. The isolation of the CS protein from a culture lysate of such host is carried out by conventional protein isolation techniques.

This invention also relates to a vaccine containing an immunoprotective amount of P. falciparum CS protein prepared by the method of this invention. By the term "immunoprotective" is meant that enough of the CS protein of this invention is administered to an infected human host to elicit sufficient protective antibody response against subsequent P. falciparum parasite infection. In the vaccine of the invention, an aqueous solution of the polypeptide of the invention, i.e., the P. falciparum CS protein expressed by the transformed yeast host cells of the invention, preferably buffered at physiological pH, can be used directly. Alternatively, the polypeptide, with or without prior lyophilization, can be admixed or absorbed with any of the various known adjuvants. Such adjuvants include, among others, aluminum hydroxide, muramyl dipeptide and saponins such as Quil A. As a further exemplary alternative, the polypeptide can be encapsulated within microparticles such as liposomes. In yet another exemplary alternative, the polypeptide can be conjugated to an immunostimulating macromolecule, such as killed Bordetella or a tetanus toxoid.

Vaccine preparation is generally described in New Trends and Developments in Vaccines, edited by Voller et al., University Park Press, Baltimore, Maryland, U.S.A., 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757. Use of Quil A is disclosed, for example, by Dalsgaard et al., Acta. Vet. Scand., Volume 18. page 349 (1977).

The amount of yeast-derived P. falciparum CS polypeptide present in each vaccine dose is selected as an amount which induces an immunoprotective response without inducing significant, adverse side effects. Such amount will vary depending upon which specific polypeptide is employed and whether or not the vaccine is adjuvanted. Generally, it is expected that each dose will comprise 1-1000 μg of polypeptide, preferably 10-200μg. An optimal amount for a particular vaccine can be ascertained by standard studies involving observations of antibody titres and other responses in subjects. Following an initial vaccination, subjects will preferably receive a boost in about 4 weeks, followed by repeat boosts every six months for as long as a risk of infection exists.

## EXAMPLES

The following Examples are illustrative, and not limiting, of the invention. All temperatures are in degrees Centigrade (Celsius). The enzymes used in DNA manipulations were obtained from Bethesda Research Laboratories, New England Biolabs and/or Boehringer, and were employed according to the suppliers directions.

In the following Examples, the following abbreviations may be employed:

YNB: Yeast Nitrogen Base without amino acids (Difco Lab) 0.675%, containing 2% glucose

PBS: Phosphate Buffered Saline (per liter):

8.0 gram NaCl

0.2 gram KCl

1.15 gram $Na_2HPO_4$        pH 7.4

0.2 gram $KH_2PO_4$

0.1 gram $CaCl_2$

0.1 gram $MgCl_2 \cdot 6H_2O$

BSA: Bovine Serum Albumin (A4378, Sigma)

L broth: (per liter) 10 gram Tryptone; 5 gram NaCl; 5 gram yeast extract; 1 ml 0.1 $\underline{M}$ $MgSO_4$. Add 5 ml of aseptic solution of thiamide (1 mg/ml) after autoclaving. For solid media, add 15 g agar per liter.

X-gal: 5-bromo-4-chloroindoyl-ß-D-galactopyranoside.

Example A. Construction of plasmid pRIT10167.

The starting material was plasmid p6y containing a 2.1 kb HindIII fragment of yeast DNA encoding the TDH3 gene cloned on pBR322. pBR322 is described by Bolivar et al., Gene, 2, 95-113 (1977). The p6y plasmid was constructed and characterized by Musti et al., Gene, 25, 133 (1983) and received from Dr. M. Rosenberg of the National Institute of Health. The HindIII fragment was recloned onto a pBR322 derivative in which the EcoRI site had been destroyed to give plasmid pRIT10164 (a non essential manouver). The following manipulations were carried out so as to create a BamHI site located at the G residue of the ATG codon of the TDH3 coding sequence and to obtain a HindIII-BamHI DNA fragment with TDH3 promoter activity in which the TDH3 sequences are intact and unchanged by the manipulation. DNA of pRIT10164, prepared as described above, was purified by two cycles of CsCl - ethidium bromide density gradient centrifugation essentially as described by Kahn et al., Methods in Enzymology, 68, 268 (1979). 150 µg of pRIT10164 DNA were digested to completion with 75 units of XbaI endonuclease, extracted with phenol and ether, precipitated with ethanol and the DNA resuspended in 0.01 M tromethamine-HCl buffer at a concentration of 1 µg per µl. Samples of 20 µg of this XbaI treated DNA were digested with Bal31 nuclease to resect the 61 base pairs of DNA between the ATG codon and the XbaI site. Digestions with Bal31 were carried out at 30° for 1 to 3 minutes in buffer containing 600 mM NaCl, 12 mM CaCl2, 12 mM MgCl2, 1 mM EDTA, 20 mM tromethamine-HCl, ph3.1 using one unit of Bal31 nuclease per 20 µg DNA in a final reaction volume of 200 µl.

The enzyme reactions were stopped by addition of ethylenebis (oxyethylenemtrilo) tetraacetic acid (EGTA) to give 20 mM final concentration and the samples were extracted with equal volumes of phenol, ether and precipitated with ethanol. Each DNA sample was resuspended in 20 µl 10 mM tromethamine-HCl buffer pH 7.5. The extent of Bal31 digestion was measured by digesting about 2.5 µg of each DNA sample with HpaI endonuclease and comparing the size of the HpaI-XbaI fragments to the 335 bp HpaI-XbaI fragment from pRIT10164. A 2 minute digestion with Bal31 was found to remove about 41 to 88 nucleotides from the XbaI-HpaI fragment of pRIT10164.

This result indicates that a similar number of base pairs had been removed from the other XbaI extremity towards the ATG codon. 5 µg of the DNA recovered from the 2 minute Bal31 digest was digested with BamHI endonuclease, extracted with phenol and recovered by ethanol precipitation. This DNA was treated with 5 units of T4 polymerase in the presence of deoxynucleotide triphosphates to fill in and render flush ended the BamHI and any Bal31 single stranded extremities, extracted with phenol and recovered by ethanol

precipitation. 2.3 μg of this DNA was treated with 5 units of T4 DNA ligase and half of the ligation mixture used to transform competent cells of E. coli K12 strain MM294 prepared according to the method of Cohen et al., Proc. Natl. Acad. Sci. 69, 2110 (1972). One ml of the transformed E. coli population was diluted into 350 ml L-broth with 200 μg/ml ampicillin and total plasmid DNA was purified from the resulting culture.

80 μg of this plasmid DNA containing a population of plasmid molecules with Bal31 induced deletions of about 40 to 80 base pairs was digested sequentially with 75 units of HindIII endonuclease and 96 units of BamHI endonuclease to release DNA fragments from those plasmids in which a BamHI site had been recreated after the treatments described above, that is, where the Bal31 digestion had terminated at a G residue. This DNA digest was run on a preparative 1% agarose gel and the desired HindIII-BamHI fragments corresponding to sizes from 1100 to 1000 bp excised from the gel in two slices, one corresponding to DNA of about 1070 bp and the other of about 1030 bp. The DNA was recovered from the two agarose gel slices by cycles of freezing and thawing followed by high speed centrifugation to pellet the agarose. The supernatant liquid was filtered through a Millipore GV Millex filter and the DNA recovered by two cycles of ethanol precipitation and resuspended in 20 μl of 0.01 M tromethamine-HCl buffer pH 7.5.

Analysis by agarose gel electrophoresis and comparison to a HindIII plus XbaI digest of pRIT10164 DNA and to the fragments from a HindIII plus EcoRI digest of phage λ DNA showed that two distinct populations of HindIII-BamHI fragments were obtained, one with an estimated size of about 1070 bp and one with an estimated size of about 1030 bp compared to the 1120 bp parental HindIII-XbaI fragment from pRIT10164. About 100 ng of the 1030 bp HindIII-BamHI fragment population was ligated with 200 ng of plasmid pUC9 which had been digested with HindIII and BamHI endonucleases and treated with alkaline phosphatase (See, Shine, U.S. Patent No. 4,264,731) and the ligation mixture was used to transform competent cells of E. coli strain JM103 with selection being made for ampicillin resistance. Transformation of E. coli strain JM103 was effected according to the method of Cohen et al., cited above.

Both the pUC9 vector plasmid and the recipient E. coli strain JM103 have been described by Vieira and Messing in Gene, 19 259 (1982) and were obtained from J. Messing (Univ. of Minnesota). The pUC9 vector is commercially available from Amersham, (Buckinghamshire, United Kingdom) and Pharmacia (Uppsala, Sweden). E. coli strain JM103 is available from J Messing (Univ. of Minnesota). Another E. coli strain with the characteristics of E. coli strain JM103, i.e., JM101, can be obtained from the American Type Culture Collection, Rockville, Maryland, under accession number ATCC 33876, and can also be employed as host. About 400 ampicillin resistant colonies per ml were obtained and of 98 colonies tested on medium containing X-gal. 95 were white indicating the successful insertion of a foreign DNA fragment between the HindIII and BamHI sites on the vector.

Plasmids from individual transformant colonies were prepared by a small scale procedure [Birnboim and Doly, Nucl. Acid Res., 7, 1513 (1979)] after amplification of the plasmids by addition of spectinomycin (150 μg/ml) to the growing cultures.

The recombinant plasmids wer analysed on 7.5% acrylamide gels after double digestion with AvaII and BamHI endonucleases and compared to the 450 bp AvaII-XbaI fragment comprising the promoter-N-terminus coding region of pRIT10164 and to the fragments of a HpaII digest of pBR322 DNA. An AvaII-BamHI fragment corresponding to deletions of from 20 to 90 base pairs were present in 35 of 36 plasmids examined when compared to pRIT10164. Three plasmids representing deletions of about 80 base pairs (pRIT10166) 50 base pairs (pRIT10167, Figure I) and 45 base pairs (pRIT10165) were chosen for further study. Plasmid DNA was prepared by CsCl-ethidium bromide density centrifugation and 25 μg of each digested with EcoRI. The EcoRI extremities were labelled with δ-$^{32}$P-ATP by exchange kination and the nucleotide sequence of the HindIII-EcoRI fragments from each plasmid determined by the chemical modification methods of Maxam and Gilbert, Methods in Enzymology, 65, 499 (1980). Labelling of and sequencing from the EcoRI site in the pUC9 vector portion of each recombinant plasmid is a convenient means of determining the sequence around the adjacent BamHI site marking the end of the deletion into the TDH3 DNA fragment. This sequencing analysis showed that in plasmid pRIT10166, the BamHI site is recreated at a G residue located in the 5′ non translated region 25 base pairs upstream of the ATG codon; in pRIT10165 the BamHI site is located at the second base of the third codon; and in pRIT10167 the BamHI site is located at the G residue of the ATG codon.

The HindIII-BamHI fragment of TDH3 DNA thus constructed in pRIT10167 contains all the sequences necessary for TDH3 promoter activity in an unaltered form.

Example B. Construction of vector pRIT10172.

The 1050 bp HindIII-BamHI TDH3 DNA insert from pRIT10167, prepared as described in Example A, was recloned onto the YEp13 shuttle vector described by Broach et al. Gene, 8, 121 (1979) between the HindIII site in the 2 micron DNA portion of the vector and the BamHI site to give the recombinant plasmid pRIT12159. DNA of pRIT12159 was then digested with XbaI and BamHI endonucleases and the resulting 1650 bp fragment containing the ARG3 promoter on plasmid pRIT10774. Plasmid pRIT10774 is described by Cabezon et al., Proc. Natl. Acad. Sci. U.S., 81, 6594-6598 (1986), as well as Cabezon et al., U.S. Patent Application Serial Number 575,122 filed January 30, 1984, and comprises the YEp13 replicon from which the natural vector's BamHI and XhoI sites have been deleted by in vitro manipulation together with a 1470 bp HindIII-BamHI insert comprising the ARG3 promoter region and a 1150 bp BamHI-HindIII frag ment comprising the ARG3 transcription termination region. Replacement of the ARG3 promoter insert on pRIT10774 by the TDH3 promoter insert gives plasmid pRIT10172 which therefore contains

6

a single BamHI site located at the ATG codon of the TDH3 promoter insert and preceeding a functional signal for transcription termination on the 1150 bp BamHI-HindIII ARG3 DNA fragment. Foreign DNA can therefore be cloned at this site. Furthermore the two DNA inserts are bounded by HindIII sites enabling removal of the expression unit module (cassette) as a 2200 bp HindIII fragment for insertion on other vectors. Figure 2 is a restriction endonuclease cleavage map of pRIT10172.

EXAMPLE I

EXPRESSION OF THE P. FALCIPARUM CIRCUMSPOROZOITE PROTEIN CODING SEQUENCE MINUS ITS PRESUMED SIGNAL SEQUENCE IN YEAST

A. Construction of Plasmids pRIT12570 and pRIT12569

Plasmid WR201 was obtained from the Walter Reed Army Institute of Research and results from insertion of a 2.3 kilobase (kb) EcoRI fragment from λmPfl [See, Dame et al., Science, 225, 593-599, (1984)]; encoding the complete circumsporozoite protein gene Plasmodium falciparum into vector pUC8. Figure 1A shows a schematic restriction map and sequencing strategy of clone λmPfl. The positions and restriction enzyme cleavage sites shown in the figure were determined from the sequence and confirmed by digestion: A, AvaII; Ac, AccI; B, BstnI; D, DraI; Dd, DdeI; F, FokI; N, NdeI; R, RsaI; S, StuI; T, TthIII; Tq, TaqI; X, XholI. Arrows indicate the origin, direction and extent of the sequences determined. The CS protein coding region is shown as a heavy line.

Figure 2A shows the nucleotide sequence of the CS protein gene from P. falciparum. The nucleotide sequence of the CS protein gene in λmPfl is shown. Vector pUC8 is described by Vieira et al., Gene, 19, 259 (1982). Vector pUC8 is commercially available from Amersham, Buckinghamshire, United Kingdom, and Pharmacia, Piscataway, New Jersey, U.S.A. and Uppsala, Sweden. A 1215 base pair (bp) StuI-RsaI fragment of plasmid WR201 containing the P. falciparum CS protein coding sequence minus the first 52 bp of the CS protein coding sequence was purified by electroelution from a 7.5% polyacrylamide electrophoresis gel. Restriction endonuclease StuI cleaves the CS protein gene in the 18th codon of the sequence. Since the sequence of the first 16 amino acids of the CS protein is characteristic of a cleaved signal peptide [Dame et al., Science, 225, 593-599 (1984)], these amino acids are presumably absent from the mature CS protein on sporozoites.

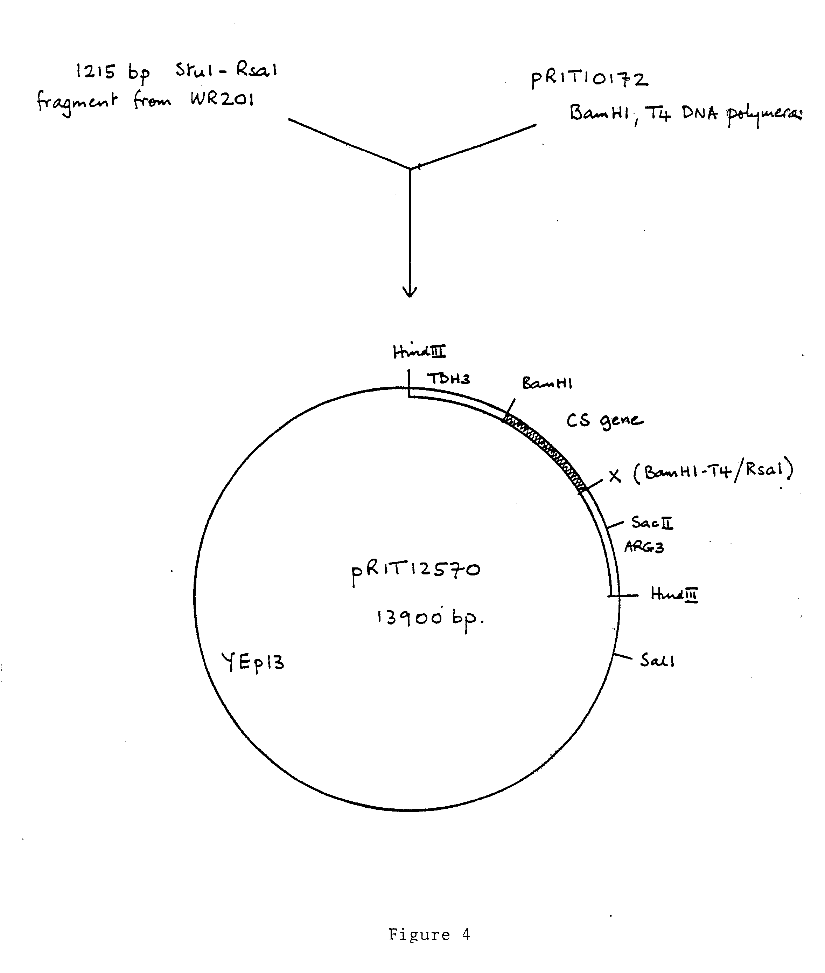

Thus, the 1215 bp StuI-RsaI fragment of plasmid WR201 should encode all but the first two amino acids of the mature CS protein predicted from the sequence. This blunt end StuI-RsaI fragment is fused, in phase, to the translation initiation codon adjacent to the DNA polymerase repaired BamHI site of yeast expression vector pRIT10172, prepared as described in Example B (the ligation of the filled-in BamHI site to the StuI site reforms the BamHI site). DNA of plasmid pRIT10172 was digested with BamHI endonuclease, treated with E. coli DNA polymerase I (Klenow fragment), extracted with phenol and recovered by ethanol precipitation. 0.4 µg of this DNA was mixed with 0.2 µg of the 1215 bp StuI-RsaI fragment from WR201 described above, treated with T$_4$ DNA ligase, and the ligation mixture was used to transform competent cells of E. coli K12 strain MM294 prepared according to the method of Cohen et al., Proc. Natl. Acad. Sci., USA, 69, 2110 (1972). 1200 colonies were tested by the method of colony hybridisation [Grunstein and Hogness, Proc. Natl. Acad. Sci., USA, 72, 3961 (1975)] on filters of Whatman 541 using the StuI-RsaI fragment, radiolabelled with $^{32}$P by nick translation, as probe. 20% of the clones gave a positive signal with the probe. 28 plasmids from positive colonies were prepared by a small scale procedure [Birnboim et al., Nucleic Acids Research, 7, 1513 (1979)]. The orientation of the StuI-RsaI fragment was tested by double digestion with BamHI and SalI endonucleases. Among the plasmids, 7 plasmids had the fragment in the correct orientation (one of which was designated pRIT12570, Figure 2) and 12 plasmids had the fragment in the wrong orientation (one of which was designated pRIT12569, Figure 2). Figure 4 is a flow chart illustrating preparation of pRIT12570.

Plasmids pRIT12570, pRIT12569, and pRIT10172 were introduced into yeast (Saccharomyces cerevisiae) strains DC5 (leu2-3, leu2-112, his3, can1-11) and 10S44C (leu2-3, leu2-112, pep4-3) obtained from M. Crabeel, Ceria Institute, Anderlecht, Belgium, by transformation of lithium acetate treated cells [(Ito et al., J. Bacteriol, 153, 163-168 (1983)] and selection for leucine independence. The protein predicted by this construction contains 394 amino acids of the mature P. falciparum CS protein fused to three amino acids (Met-Asp-Pro-) at its NH$_2$-terminus which are derived from the vector (pRIT10172) sequence. S. cerevisiae strain 10S44C was deposited at the American Type Culture Collection, Rockville, Maryland, U.S.A., in accordance with the regulations of the Budapest Treaty, on August 18, 1986, under accession number ATCC 20819. S. cerevisiae strain DC5 was deposited at the American Type Culture Collection on June 2, 1982, in accordance with the regulations of the Budapest Treaty, under accession number ATCC 20630.

B. Synthesis of P. falciparum Circumsporozoite Protein in Yeast.

Yeast (Saccharomyces cerevisiae) strains DC5 or 10S44C containing either pRIT12570, pRIT12569 or pRIT10172, prepared as described above, were separately grown in liquid medium lacking leucine (YNB + 80 µg/ml histidine or YNB). CS protein sequences were identified by protein immunoblotting technique [See, Burnette, Anal. Biochem., 112, 195-203 (1981); Gershoni and Palade, Anal. Biochem., 131, 1-15 (1983); Towbin and Gordon, J. Immunol. Methods, 72, 313-340 (1984)] using a mixture of 5 monoclonal antibodies against CS protein [[Dame et al., Science, 225, 593-599 (1984)]. After electroblotting of the proteins onto a nitrocellulose filter (Schleicher and Schüll, 0,45 u) [Towbin et al., Proc. Natl. Acad. Sci., U.S.A., 76, 4350-4354, (1979)], the filter was preincubated for one hour at 37° with 3% gelatin in PBS. Subsequently the filter was washed 5 times for 5 min. each time with PBS containing 0.1% Tween 20 (polyoxyethylene sorbitan monolaurate), and the sheet was treated for one hour at room temperature with a mixture of five monoclonal antibodies against CS protein [Dame et al., Science 225, 593-599, (1984)] in PBS, 1% gelatin. The filter sheets were washed 5 times for 5 min. each time with PBS, 0.1% Tween 20, and a second biotinylated sheep anti-mouse antibody (Amersham) in PBS, 1% gelatin was added for one hour at room temperature. The sheets were washed again with PBS, 0.1% Tween 20, 5 times for 5 min. each time, and were incubated for 30 min. at room temperature with streptavidin-biotinylated horseradish peroxidase (HRP) complex (Amersham). The sheets were washed again 3 times for 5 min. each time with PBS, 0.1% Tween 20, and were finally incubated with 30 μl $H_2O_2$ and HRP Color Development Reagent containing 4-chloro-1-naphtol (BioRad), 30 mg in 30 ml methanol, in 50 ml PBS. The molecular weight of the antigens was estimated from prestained protein markers, ovalbumin, alpha-chymotrypsinogen, beta-lactoglobin, lysozyme, cytochrome C (BRL).

The immune reaction specific for proteins derived from pRIT12570 containing cells showed protein species ranging in molecular weight from 30,000 to 50,000 daltons. This shows that the P. falciparum CS related product expressed by yeast is heterogeneous. More of this heterogeneous material was found among the proteins from 10S44C transformed cells than from DC5 transformed cells.

EXAMPLE II

Antibody Response - ELISA, CS Reactivity of antisera to yeast prepared P. falciparum CS protein, Hepatocyte Blocking

The P. falciparum CS polypeptide prepared by yeast according to the method of this invention can be evaluated for its immunogenicity according to the methods outlined in Ballou et al., U.S. Patent Application Serial Number 699,116, filed February 7, 1985, the entire disclosure of which is hereby incorporated by reference.

EXAMPLE III

Vaccine Containing P. Falciparum CS Protein Expressed By Yeast

A illustrative vaccine of this invention is prepared as follows: To a buffered, aqueous solution of 3% aluminum hydroxide (10 mM sodium phosphate, 150 mM NaCl, pH 6.8; sterilized by filtration), the yeast derived P. falciparum CS polypeptide of this invention in similar buffer is added with stirring to a final concentration of 100 ug/ml of polypeptide and 0.5 mg/ml of aluminum ($Al^{3+}$). The pH is maintained at 6.8. The mixture is left overnight at about 0°. Thimerosal is added to a final concentration of 0.0005%. The pH is checked and adjusted, if necessary, to 6.8.

EXAMPLE IV

CONSTRUCTION OF VECTORS FOR THE EXPRESSION OF IMMUNOGENIC PORTIONS OF THE TETRAPEPTIDE REPEAT REGION OF THE P. FALCIPARUM CS PROTEIN IN YEAST

A. Construction of pRIT12290.

A 1050 bp HindIII-EcoRI fragment from pRIT10167 (Figure I), prepared as described in Example A, and containing the HindIII-BamHI TDH3 promoter fragment together with the BamHI-SmaI-EcoRI part of the pUC9 polylinker was recloned between the HindIII and EcoRI sites of a pBR322 derivative plasmid previously deleted fro the BamHI site by filling in with T4 DNA polymerase and religation. The resulting recombinant plasmid was identified as pRIT12176.

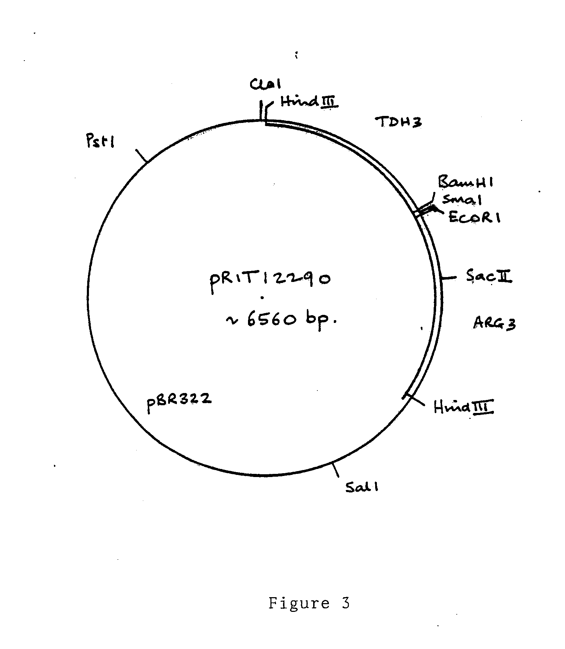

Plasmid DNA of pRIT 10158 (Figure I), prepared as described in Example V, infra, was digested with EcoRI endonuclease and treated with T4 DNA polymerase to fill in the EcoRI single strand extensions. This preparation was further digested with ClaI endonuclease. A further sample of pRIT10158 DNA was digested with ClaI and HaeIII endonucleases and a 1150 bp ClaI-HaeIII fragment carrying the transcription termination region of the ARG3 gene was recovered by preparative agarose gel electrophoresis and electroelution. This fragment was ligated with the EcoRI, T4 DNA polymerase, ClaI treated pRIT10158 DNA, and the ligation mixture was used to transform competent cells of E. coli strain MM294 prepared as described by Cohen et al., above, to ampicillin resistance. From the transformed colonies, a plasmid, identified as pRIT10162, was isolated in which the ClaI-HaeIII ARG3 transcription termination fragment was inserted on pRIT10158 between the ClaI and filled in EcoRI sites and in which the EcoRI site has been recreated. Figure 1 is a flow sheet which illustrates the preparation of pRIT10162. Plasmid DNA of pRIT10162 was digested with EcoRI and PstI endonucleases, mixed with plasmid DNA of pRIT12176, prepared as described above, (also digested with EcoRI and PstI endonucleases) and the mixture was ligated and used to transform cells of E. coli K12 strain MM294 to ampicillin resistance by the methods of Cohen et al., cited above. A plasmid, pRIT12208, was recovered from a colony from this transformation in which the large 4630 bp EcoRI-PstI fragment from pRIT12176 had been ligated to the small 1930 bp EcoRI-PstI fragment from pRIT10162, and in which the ARG3 transcription termination region is now juxtaposed to the TDH3 promoter. The ARG3 and TDH3 DNA regions can be excised from pRIT12208 as a single 2200 bp fragment by digestion with HindIII endonuclease. To obtain the other orientation of this fragment with respect to vector sequences, the 2200 bp HindIII fragment from pRIT12208 was recloned into the HindIII site of a pBR322 derivative plasmid in which the natural EcoRI and BamHI sites had separately been deleted by filling in with T4 DNA polymerase and religation. The resulting plasmid, with the 2200 bp HindIII fragment inserted in the opposite orientation with respect to vector sequences compared to pRIT12208, is identified as pRIT12290. Figure 3 is a restriction endonuclease cleavage map of pRIT12290. In both the pRIT12208 and pRIT12290 vectors, the TDH3 promoter and ARG3 transcription termination fragments are separated by unique BamHI, SmaI and EcoRI restriction sites useful for the cloning of DNA fragments carrying coding sequences, and the promoter and transcription termination regions can be excised together on a 2200 bp HindIII fragment as a module or cassette for transfer to other vectors.

The BamHI site is especially useful as it is located at the ATG codon of the TDH3 gene and can be employed for fusion of other genes to the TDH3 promoter with a view to expressing them in yeast as exemplified below. Such fusions can be recovered from pRIT12208 or pRIT12290 derivatives in the form of cassettes or modules for insertion on yeast shuttle vectors by digestion with HindIII endonuclease or by the use of other restriction endonucleases cutting at flanking restriction sites.

B. Construction of a Plasmid Containing a Synthetic Linker for Introduction of a Portion of the P. falciparum CS Protein Tetrapeptide Repeat Region

Plasmid pRIT12290, prepared as described in part A, above, is digested with BamHI and EcoRI endonucleases and mixed with a synthetic DNA fragment having the following composition

5'      GATCCTTAAG      3'

         GAATTCTTAA

The mixture of fragments is annealed and ligated with T4 DNA ligase and used to transform competent cells of E. coli strain MM294 to ampicillin resistance according to conventional techniques. Plasmids prepared from single colonies are examined for the presence of single EcoRI and BamHI endonuclease restriction sites and for the absence of a SmaI site which is present on the original pRIT12290 vector but not on the desired recombinant. The correctness of the plasmid chosen can be verified by DNA sequencing. Insertion of the synthetic DNA fragment recreates a BamHI site following the TDH3 ATG codon and provides a stop codon (TAA) in phase with the ATG codon. The purpose of creating such a plasmid is to create a suitable vector for fusion of the 192 base pair portion of the P. falciparum CS protein tetrapeptide repeat region to the TDH₃ promoter region and provide a stop codon immediately 3' to such coding region.

C. Construction of a Plasmid Containing a 192 Base Pair Portion of the P. falciparum CS Protein Tetrapeptide Repeat Region

The 1215bp StuI-RsaI fragment of plasmid WR201, purified as described in Example I, and containing the CS protein coding sequence minus the first 52 bp is digested with Sau3A to generate smaller fragments which include, among others, a 192 bp Sau3A fragment coding for 16 tetrapeptide repeats of the CS protein. The BamHI site of the modified pRIT12290 vector of part B, above, located at the ATG initiation codon, is in phase with Sau3A sites of the repetitive epitope of the CS protein of P. falciparum.

DNA of this plasmid is digested with BamHI endonuclease, extracted with phenol and recovered by ethanol precipitation. 0.2 µg of this DNA is mixed with about 0.1 to 0.2 µg of the Sau3A digest of the StuI-RsaI fragment, prepared as described above, treated with T4 DNA ligase and the ligation mixture used to transform competent cells of E. coli strain MM294 to ampicillin resistance according to conventional techniques. Plasmids from individual transformants are prepared and analyzed on 7.5% acrylamide gels after double digestion with BamHI and EcoRI endonucleases and compared to the parent vector, to the Sau3A digest of the StuI-RsaI CS gene fragment, and to suitable molecular weight markers e.g. a HaeIII digest of Øx174 DNA.

Plasmids containing a BamHI-EcoRI insert fragment of 200 bp correspond to the insertion of the 192 bp Sau3A fragment in the desired orientation, for fusion of the CS coding sequence to the ATG initiation codon. Insertion of the 192 bp Sau3A fragment in the desired orientation recreates the BamHI site at the ATG codon.

In addition, digestion of such a plasmid with Sau3A will liberate a 192 bp fragment of the same size as that obtained from digestion of the StuI-RsaI fragment containing the CS protein repetitive epitope. This construction will contain an open reading frame of 67 amino acids extending from the ATG codon at the TDH3 promoter boundary through the CS repetitive epitope insert and terminating at the TAA codon in the introduced synthetic DNA fragment immediately 3' to the Sau3A site and 5' to the EcoRI site. Correctness of the construct can be verified by DNA sequencing, and digestion of the plasmid with HindIII endonuclease will liberate a HindIII fragment of 2390 bp comprising the TDH3 promoter, the CS repetitive epitope and the ARG3 termination region. This HindIII fragment can be recloned onto any suitable yeast vector, e.g., YEp13, and introduced into yeast according to conventional techniques.

The plasmid can also be digested with BamHI endonuclease and mixed and ligated again with the Sau3A digest of the StuI-RsaI CS repetitive epitope fragment to introduce a tandem copy of the epitope which will be in phase with the first copy, and as before will recreate a BamHI site at the ATG codon provided the orientation of the insert is correct. This process may be repeated to build up a series of repeats of the 192 bp epitope

# 0 278 941

fragment comprising two, three, four etc. tandem copies. This process will increase the length of the open reading frame and result in progressively larger proteins comprising 131, 195 and 259 amino acids.

Digestion of these constructs with HindIII endonuclease will liberate progressively larger fragments containing the expression cassettes which may be recloned onto suitable yeast vectors for transformation and introduction into yeast by known methods such as those described in Example I.

## EXAMPLE V

### Construction of Plasmid pRIT10158

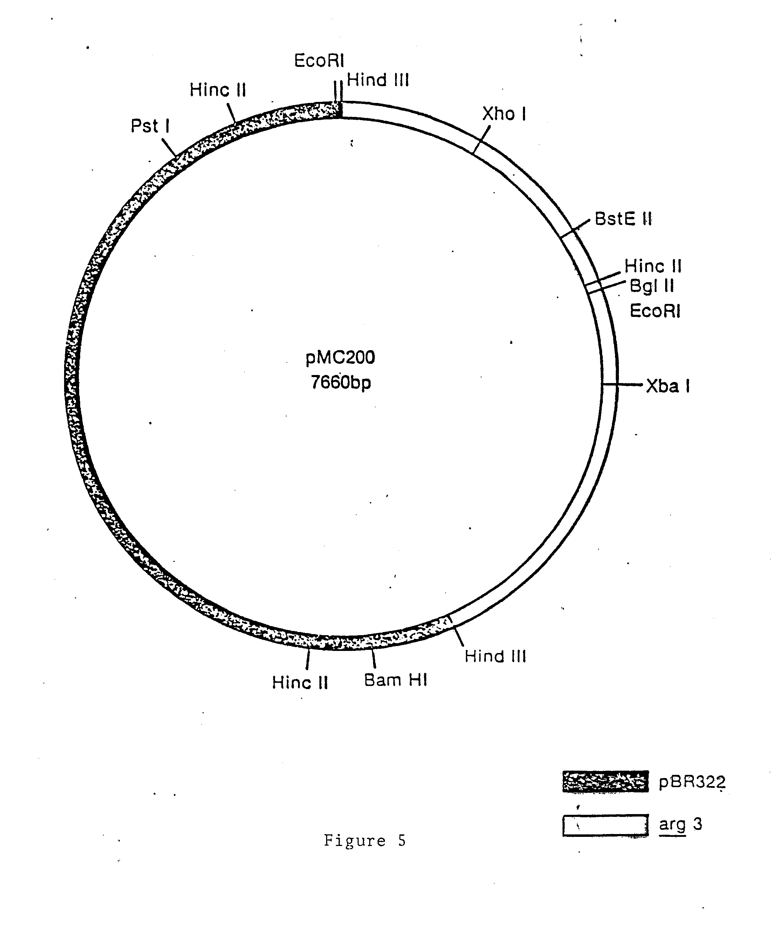

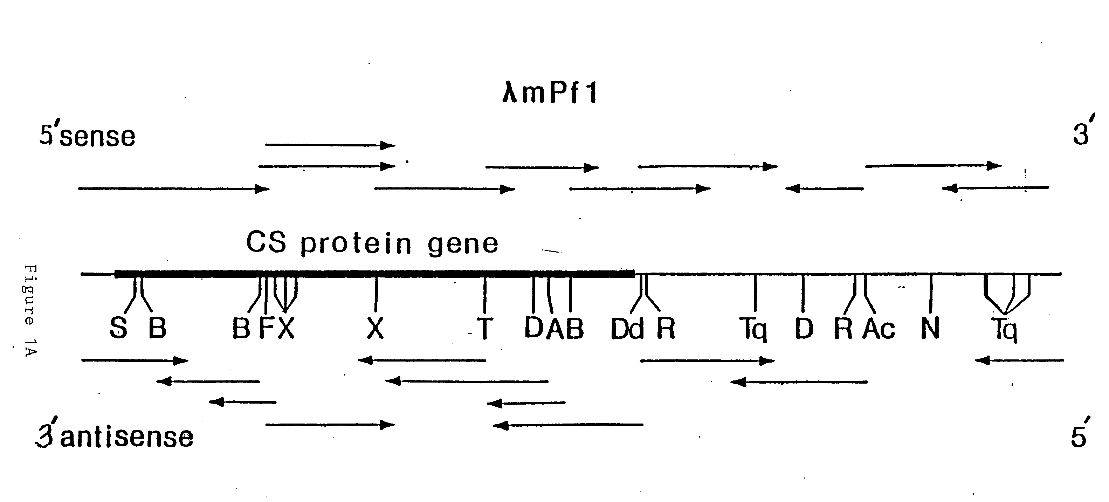

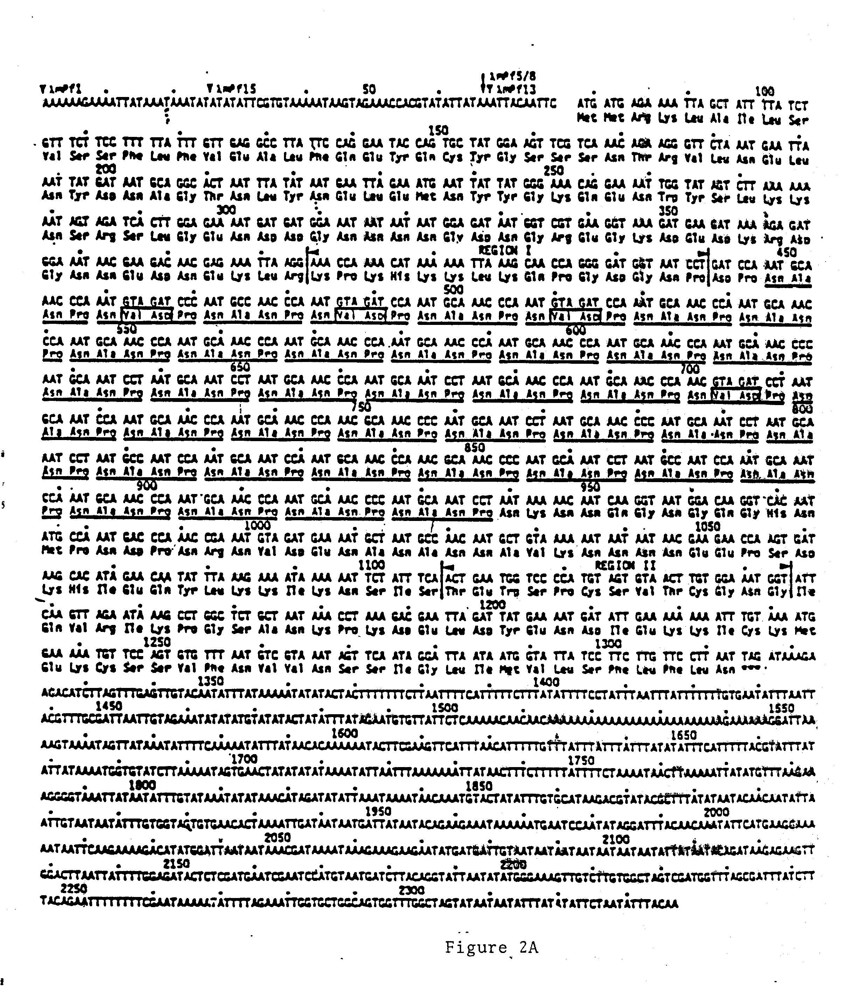

The 3300 bp HindIII fragment from plasmid pMC200 (available from the American Type Culture Collection, Rockville, Maryland, U.S.A., without restriction under accession number ATCC 39131),described by Crabeel M. et al; EMBO J., 2, 205-212 (1983), was recloned onto a pBR322 derivative plasmid on which the natural EcoRI site had been deleted by filling in with T4 DNA polymerase and religation. A resulting recombinant plasmid, pRIT10158 (Figure I), was selected in which the HindIII ARG3 gene insert has the 3' region of the ARG3 gene proximal to the ClaI site on the modified pBR322 vector. Figure 5 is a restriction endonuclease cleavage map of pMC200.

While the above fully describes the invention and all preferred embodiments thereof, it is to be appreciated that the invention is not limited to the embodiments particularly described but rather includes all modifications thereof coming within the scope of the following claims.

## Claims

1. A recombinant DNA vector comprising a DNA sequence operatively linked to an expression control sequence, wherein the DNA sequence contains the coding sequence of the circumsporozoite protein of Plasmodium falciparum.

2. The vector of Claim 1 which additionally comprises a replicon which is functional in yeast.

3. The vector of Claim 1 in which the expression control sequence comprises the yeast glyceraldehyde-3P-dehydrogenase (TDH3) promoter.

4. The vector of Claim 3 in which the expression control sequence additionally comprises the ARG3 transcription termination region.

5. The vector of Claim 4 which comprises a 1215 bp StuI-RsaI fragment of WR201 containing the P. falciparum CS protein coding sequence minus approximately its first 50 bp.

6. The vector of Claim 5 which is pRIT12570.

7. The vector of Claim 4 which comprises a 192 bp Sau3A fragment coding for 16 tetrapeptidie repeats of the P. falciparum CS protein derived from Sau3A digestion of a 1215 bp StuI-RsaI fragment of WR201 containing the P. falciparum CS protein coding sequence minus approximately its first 50 bp, or two, three, four or more tandem copies of such 192 bp Sau3A fragment.

8. A yeast host cell transformed with a recombinant DNA vector, wherein said vector comprises a DNA sequence operatively linked to an expression control sequence, wherein the DNA sequence contains the coding sequence of the circumsporozoite protein of Plasmodium falciparum.

9. The host of Claim 8 wherein the vector additionally comprises a replicon which is functional in yeast.

10. The host of Claim 8 wherein the expression control sequence comprises the yeast glyceraldehyde-3P-dehydrogenase (TDH3) promoter.

11. The host of Claim 10 wherein the expression-control sequence additionally comprises the ARG3 transcription termination region.

12. The host of Claim 11 wherein the vector comprises a 1215 bp StuI-RsaI fragment of WR201 containing the P. falciparum CS protein coding sequence minus, approximately its first 50 bp.

13. The host of Claim 12 wherein the vector is pRIT12570.

14. The host of Claim 11 wherein the vector comprises a 192 bp Sau3A fragment coding for 16 tetrapeptide repeats of the P. falciparum CS protein derived from Sau3A digestion of a 1215 bp StuI-RsaI fragment of WR201 containing the P. falciparum CS protein coding sequence minus approximately its first 50 bp, or two, three, four or more tandem copies of such 192 bp Sau3A fragment.

15. The host of Claim 8 which belongs to the genus Saccharomyces.

16. The host of Claim 15 which belongs to the species S. cerevisiae.

17. The host of Claim 16 which is S. cerevisiae strain DC5 or 10S44C.

18. A method of preparing a yeast host cell transformed with a recombinant DNA vector, wherein said vector comprises a DNA sequence operatively linked to an expression control sequence, wherein the DNA sequence contains the coding sequence of the circumsporozoite protein of Plasmodium falciparum.

19. The method of Claim 18 wherein the vector additionally comprises a replicon which is functional in yeast.

20. The method of Claim 18 wherein the expression control sequence comprises the yeast glyceraldehyde-3P-dehydrogenase (TDH3) promoter.

21. The method of Claim 20 wherein the expression control sequence additionally comprises the ARG3 transcription termination region.

22. The method of Claim 21 wherein the vector comprises a 1215 bp StuI-RsaI fragment of WR201 containing the P. falciparum CS protein coding sequence minus approximately its first 50 bp.

23. The method of Claim 22 wherein the vector is pRIT12570.

24. The method of Claim 21 wherein the vector comprises a 192 bp Sau3A fragment coding for 16 tetrapeptide repeats of the P. falciparum CS protein derived from Sau3A digestion of a 1215 bp StuI-RsaI fragment of WR201 containing the P. falciparum CS protein coding sequence minus approximately its first 50 bp, or two, three, four or more tandem copies of such 192 bp Sau3A fragment.

25. The method of Claim 18 wherein the host belongs to the genus Saccharomyces.

26. The method of Claim 25 wherein the host belongs to the species S. cerevisiae.

27. The method of Claim 26 wherein the host is S. cerevisiae strain DC5 or 10S44C.

28. A method for preparing circumsporozoite protein of Plasmodium falciparum, which comprises culturing a yeast hose cell transformed with a recombinant DNA vector in appropriate culture media and isolating such protein from a culture lysate of such host, wherein said vector comprises a DNA sequence operati vely linked to an expression control sequence, wherein the DNA sequence contains the coding sequence of the circumsporozoite protein of Plasmodium falciparum.

29. The method of Claim 28 wherein the vector additionally comprises a replicon which is functional in yeast.

30. The method of Claim 28 wherein the expression control sequence comprises the yeast glyceraldehyde-3P-dehydrogenase (TDH3) promoter.

31. The method of Claim 30 wherein the expression control sequence additionally comprises the ARG3 transcription termination region.

32. The method of Claim 31 wherein the vector comprises a 1215 bp StuI-RsaI fragment of WR201 containing the P. falciparum CS protein coding sequence minus approximately its first 50 bp.

33. The method of Claim 32 wherein the vector is pRIT12570.

34. The method of Claim 31 wherein the vector comprises a 192 bp Sau3A fragment coding for 16 tetrapeptide repeats of the P. falciparum CS protein derived from Sau3A digestion of a 1215 bp StuI-RsaI fragment of WR201 containing the P. falciparum CS protein coding sequence minus approximately its first 50 bp.

35. The method of Claim 28 wherein the host belongs to the genus Saccharomyces.

36. The method of Claim 35 wherein the host belongs to the species S. cerevisiae.

37. The method of Claim 36 wherein the host is S. cerevisiae strain DC5 or 10S44C.

38. The protein prepared according to the method of Claim 26, 27, 28, 29, 30, 31, 32 or 33.

39. A vaccine comprising an immunoprotective quantity of the protein of Claim 38.

40. The vaccine of Claim 39 which comprises 1-1000 mg of the protein.

41. The vaccine of Claim 40 which comprises 10-200 mg of the protein.


Claims for the following contracting States : ES, GR

1. A method of preparing a yeast host cell transformed with a recombinant DNA vector, wherein said vector comprises a DNA sequence operatively linked to an expression control sequence, wherein the DNA sequence contains the coding sequence of the circumsporozoite protein of Plasmodium falciparum.

2. The method of Claim 1 wherein the vector additionally comprises a replicon which is functional in yeast.

3. The method of Claim 1 wherein the expression control sequence comprises the yeast glyceraldehyde-3P-dehydrogenase (TDH3) promoter.

4. The method of Claim 3 wherein the expression control sequence additionally comprises the ARG3 transcription termination region.

5. The method of Claim 4 wherein the vector comprises a 1215 bp StuI-RsaI fragment of WR201 containing the P. falciparum CS protein coding sequence minus approximately its first 50 bp.

6. The method of Claim 5 wherein the vector is pRIT12570.

7. The method of Claim 4 wherein the vector comprises a 192 bp Sau3A fragment coding for 16 tretrapeptide repeats of the P. falciparum CS protein derived from Sau3A digestion of a 1215 bp StuI-RsaI fragment of WR201 containing the P. falciparum CS protein coding sequence minus approximately its first 50 bp, or two, three, four or more tandem copies of such 192 bp Sau3A fragment.

8. The method of Claim 3 wherein the host belongs to the genus Saccharomyces.

9. The method of Claim 8 wherein the host belongs to the species Sp cerevisiae.

10. The method of Claim 9 wherein the host is S. cerevisiae strain DC5 or 10S44C.

11. A method for preparing circumsporozoite protein of Plasmodium falciparum, which comprises culturing a yeast host cell transformed with a recombinant DNA vector in appropriate culture media and isolating such protein from a culture lysate of such host, wherein said vector comprises a DNA sequence operatively linked to an expression control sequence, wherein the DNA sequence contains the coding sequence of the circumsporozoite protein of Plasmodium falciparum.

12. The method of Claim 11 wherein the vector additionally comprises a replicon which is functional in yeast.

13. The method of Claim 11 wherein the expression control sequence comprises the yeast glyceraldehyde-3P-dehydrogenase (TDH3) promoter.

14. The method of Claim 13 wherein the expression control sequence additionally comprises the ARG3 transcription termination region.

15. The method of Claim 14 wherein the vector comprises a 1215 by Stul-Rsal fragment of WR201 containing the P. falciparum CS protein coding sequence minus approximately its first 50 bp.

16. The method of Claim 15 wherein the vector is pRIT12570.

17. The method of Claim 14 wherein the vector comprises a 192 bp Sau3A fragment coding for 16 tetrapeptide repeats of the P. falciparum CS protein derived from Sau3A digestion of a 1215 bp Stul-Rsal fragment of WR201 containing the P. falciparum CS protein coding sequence minus approximately its first 50 bp.

18. The method of Claim 11 wherein the host belongs to the genus Saccharomyces.

19. The method of Claim 18 wherein the host belongs to the species S. cerevisiae.

20. The method of Claim 19 wherein the host is S. cerevisiae strain DC5 or 10S44C.

0278941

**TDH 3 promoter fragment cloned on pUC 9. BamH I site at ATG (ATGGATCC)**

pRIT 10167
3750 bp
H
E
B

Restriction Enzyme Symbols

| | | |
|---|---|---|
| A....Acc I | E....EcoR I | S....Sal I |
| B....BamH I | H....Hind III | X....Xba I |
| C....Cla I | Hp..Hpa I | Xh..Xno I |
| vector sequences | ——— | |
| cloned fragments | ⊏⊐ | |

Reclone 3300 bp ARG 3 gene
fragment from pMC 200 on
pBR 322 deleted for EcoR I site

pRIT 10158
7660 bp
C H
Hae III
X
E
Xh
B H

clone 1150 bp Cla I Hae III
fragment on EcoR I,
T 4 DNA polymerase,
Cla I treated plasmid

pRIT 10162
7000 bp
C H
E
Xh
H

Figure 1

Figure 2

0278941

Figure 3

1215 bp  StuI - RsaI
fragment from WR201

pRIT10172

BamHI, T4 DNA polymeras

Figure 4

0278941

Figure 5

Figure 1A

0278941

```
▼Vamp1            ▼Vamp15              50        ▼Vamp15/8                              100
AAAAAGAAAATTATAAATAAATATATATATTCGTGTAAAAATAAGTAGAAACCACGTATATTATAAATTACAATTC  ATG ATG AGA AAA TTA GCT ATT TTA TCT
                                                         ▼Vamp13              Met Met Arg Lys Leu Ala Ile Leu Ser

                                                            150
GTT TCT TCC TTT TTA TTT GTT GAG GCC TTA TTC CAG GAA TAC CAG TGC TAT GGA AGT TCG TCA AAC AGA AGG GTT CTA AAT GAA TTA
Val Ser Ser Phe Leu Phe Val Glu Ala Leu Phe Gln Glu Tyr Gln Cys Tyr Gly Ser Ser Ser Asn Thr Arg Val Leu Asn Glu Leu
   200                                                                      250
AAT TAT GAT AAT GCA GGC ACT AAT TTA TAT AAT GAA TTA GAA ATG AAT TAT TAT GGG AAA CAG GAA AAT TGG TAT AGT CTT AAA AAA
Asn Tyr Asp Asn Ala Gly Thr Asn Leu Tyr Asn Glu Leu Glu Met Asn Tyr Tyr Gly Lys Gln Glu Asn Trp Tyr Ser Leu Lys Lys
        300                                                          350
AAT AGT AGA TCA CTT GGA GAA AAT GAT GAT GGA AAT AAT AAT AAT GGA GAT AAT GGT CGT GAA GGT AAA GAT GAA GAT AAA AGA GAT
Asn Ser Arg Ser Leu Gly Glu Asn Asp Asp Gly Asn Asn Asn Asn Gly Asp Asn Gly Arg Glu Gly Lys Asp Glu Asp Lys Arg Asp
                                                            REGION I                                           450
GGA AAT AAC GAA GAC AAC GAG AAA TTA AGG|AAA CCA AAA CAT AAA AAA TTA AAG CAA CCA GGG GAT GGT AAT CCT|GAT CCA AAT GCA
Gly Asn Asn Glu Asp Asn Glu Lys Leu Arg|Lys Pro Lys His Lys Lys Leu Lys Gln Pro Gly Asp Gly Asn Pro|Asp Pro Asn Ala
                                                    500
AAC CCA AAT GTA GAT CCC AAT GCC AAC CCA AAT GTA GAT CCA AAT GCA AAC CCA AAT GTA GAT CCA AAT GCA AAC CCA AAT GCA AAC
Asn Pro Asn Val Asp Pro Asn Ala Asn Pro Asn Val Asp Pro Asn Ala Asn Pro Asn Val Asp Pro Asn Ala Asn Pro Asn Ala Asn
   550                                                              600
CCA AAT GCA AAC CCA AAT GCA AAC CCA AAT GCA AAC CCA AAT GCA AAC CCA AAT GCA AAC CCA AAT GCA AAC CCA AAT GCA AAC CCC
Pro Asn Ala Asn Pro Asn Ala Asn Pro Asn Ala Asn Pro Asn Ala Asn Pro Asn Ala Asn Pro Asn Ala Asn Pro Asn Ala Asn Pro
                              650                                                        700
AAT GCA AAT CCT AAT GCA AAT CCT AAT GCA AAC CCA AAT GCA AAT CCT AAT GCA AAC CCA AAT GCA AAC CCA AAC GTA GAT CCT AAT
Asn Ala Asn Pro Asn Ala Asn Pro Asn Ala Asn Pro Asn Ala Asn Pro Asn Ala Asn Pro Asn Ala Asn Pro Asn Val Asp Pro Asn
                                            750                                                              800
GCA AAT CCA AAT GCA AAC CCA AAT GCA AAC CCA AAT GCA AAC CCC AAT GCA AAT CCT AAT GCA AAC CCC AAT GCA AAT CCT AAT GCA
Ala Asn Pro Asn Ala Asn Pro Asn Ala Asn Pro Asn Ala Asn Pro Asn Ala Asn Pro Asn Ala Asn Pro Asn Ala Asn Pro Asn Ala
                                                    850
AAT CCT AAT GCC AAT CCA AAT GCA AAT CCA AAT GCA AAC CCA AAC GCA AAC CCC AAT GCA AAT CCT AAT GCC AAT CCA AAT GCA AAT
Asn Pro Asn Ala Asn Pro Asn Ala Asn Pro Asn Ala Asn Pro Asn Ala Asn Pro Asn Ala Asn Pro Asn Ala Asn Pro Asn Ala Asn
            900                                                            950
CCA AAT GCA AAC CCA AAT GCA AAC CCA AAT GCA AAC CCC AAT GCA AAT CCT AAT AAA AAC CAA GGT AAT GGA CAA GGT CAC AAT
Pro Asn Ala Asn Pro Asn Ala Asn Pro Asn Ala Asn Pro Asn Ala Asn Pro Asn Lys Asn Asn Gln Gly Asn Gly Gln Gly His Asn
                                    1000                                                        1050
ATG CCA AAT GAC CCA AAC CGA AAT GTA GAT GAA AAT GCT AAT GCC AAC AAT GCT GTA AAA AAT AAT AAT AAC GAA GAA CCA AGT GAT
Met Pro Asn Asp Pro Asn Arg Asn Val Asp Glu Asn Ala Asn Ala Asn Asn Ala Val Lys Asn Asn Asn Asn Glu Glu Pro Ser Asp
                                    1100                                     REGION II
AAG CAC ATA GAA CAA TAT TTA AAG AAA ATA AAA AAT TCT ATT TCA|ACT GAA TGG TCC CCA TGT AGT GTA ACT TGT GGA AAT GGT|ATT
Lys His Ile Glu Gln Tyr Leu Lys Lys Ile Lys Asn Ser Ile Ser|Thr Glu Trp Ser Pro Cys Ser Val Thr Cys Gly Asn Gly|Ile
                                                        1200
CAA GTT AGA ATA AAG CCT GGC TCT GCT AAT AAA CCT AAA GAC GAA TTA GAT TAT GAA AAT GAT ATT GAA AAA AAA ATT TGT AAA ATG
Gln Val Arg Ile Lys Pro Gly Ser Ala Asn Lys Pro Lys Asp Glu Leu Asp Tyr Glu Asn Asp Ile Glu Lys Lys Ile Cys Lys Met
            1250                                                        1300
GAA AAA TGT TCC AGT GTG TTT AAT GTC GTA AAT AGT TCA ATA GGA TTA ATA ATG GTA TTA TCC TTC TTG TTC CTT AAT TAG ATAAAGA
Glu Lys Cys Ser Ser Val Phe Asn Val Val Asn Ser Ser Ile Gly Leu Ile Met Val Leu Ser Phe Leu Phe Leu Asn ***
                        1350                                                1400
ACACATCTTAGTTTGAGTTGTACAATATTTTATAAAAATATATACTACTTTTTTTGTTAATTTTCATTTTTGTTTATATTTCCTATTTAATTTATTTTTTTGTGAATATTTAATT
            1450                                                1500                                                1550
ACCGTTTGCCGATTAATTGTAGAAATATATATGTATATACTATATTTATAGAATGTGTTATTCTCAAAACAACACAAAAAAAAAAAAAAAAAAAAAAGAAAAAGGATTAA
                        1600                                                    1650
AAGTAAAATAGTTATAAATATTTTCAAAAATATTTATAACACAAAAATACTTCGAAGTTCATTTAACATTTTTGTTTATTTATTTATATATTTCATTTTTACGTATTTAT
            1700                                                1750
ATTATAAAATGGTGTATCTTAAAAATAGTCAACTATATATATAAAATATTAATTTAAAAAAATTATAACTTTCTTTTTATTTTCTAAAATAACTTAAAAATTATATGTTTAAGAA
        1800                                                1850
AGGGGTAAATTATAATATTTGTATAAATATATAAACATAGATATATTAAATAAATAACAAATGTACTATATTTGTGCATAAGACGTATACGGTTTATATAATACAACAATATTA
            1900                                                    1950                                                2000
ATTGTAATAATATTTGTGGTAGTGTGAACACTAAAATTGATAATAATGATTATAATACAGAGAAATAAAAATGAATCCAATATAGGATTTACAACAAATATTCATGAAGGAAA
                1950                                                2050                                                2100
AATAATTCAACAAAGACATATGGATTAATAATAACGATAAATAAGAAAGAAGAATATGATGATTGTAATAATAATAATAATAATATTTATAATAACAGATAACAGAAGTT
                2150                                                    2200
GGACTTAATTATTTTGGAGATACTCTCGATGAATCGAATCCATGTAATGATCTTACAGGTATTAATATATGGGAAAGTTGTCTTGTGGCTAGTCGATCGGTTTAGCGATTTATCTT
            2250                                                2300
TACAGAATTTTTTTTTCGAATAAAAATATTTTAGAAATTCGTGCTCGCCAGTCGTTTGGCTAGTATAATAATATTTATATATTCTAATATTTACAA
```

Figure 2A

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 175 261 (CHIRON CORP.)<br>* Pages 41-46 * | 1-3,8-10,15-20,25-30,35-41 | C 12 N 15/00<br>C 12 N 1/18<br>C 12 P 21/02<br>C 07 K 13/00<br>A 61 K 39/015 |
| Y | | 4,11,21,31. | |
| Y | EP-A-0 151 102 (SMITH KLINE-RIT SA)<br>* Page 11, paragraph 1 * | 4,11,21,31 | |
| A | EP-A-0 192 626 (SMITH KLINE-BECKMAN CORP.)<br>* Pages 1-5 * | 1-41 | |

TECHNICAL FIELDS
SEARCHED (Int. Cl.4)

C 12 N
C 12 P
A 61 K

The present search report has been drawn up for all claims

| Place of search | Date of completion of the search | Examiner |
|---|---|---|
| THE HAGUE | 31-03-1988 | CUPIDO M. |